# EUROPEAN PATENT SPECIFICATION

(11) **EP 4 632 375 B1**
(45) Date of publication and mention of the grant of the patent: **14.01.2026**
(21) Application number: 24181625.5
(22) Date of filing: 12.06.2024
(51) Int. Cl.: G01N 33/205

(54) **MEASURING PROBE FOR MOLTEN METAL**
MESSSONDE FÜR METALLSCHMELZEN
SONDE DE MESURE POUR MÉTAL EN FUSION

(30) Priority: 12.04.2024 EP 24169906
(43) Date of publication of application: 15.10.2025
(73) Proprietor: Heraeus Electro-Nite International N.V., 3530 Houthalen (BE)
(72) Inventor: Neyens, Guido, 3530 Houthalen (BE); Bex, Gert-Jan, 3530 Houthalen (BE); Stevens, Frank, 3530 Houthalen (BE)
(74) Representative: Heraeus IP

(56) References cited:
- EP-A1- 0 997 716
- DE-A1- 102006 005 476

## Description

The present invention relates to a measuring probe for determining at least one parameter of a molten metal. In a further aspect, the invention relates to a method to measure the at least one parameter of a metal melt.

Especially during the metal making process employed in the steel industry, several parameters of the metal melt are critical for the control of the metallurgical process, for example the bath chemistry or the temperature of such a melt. The ability to continuously and/or periodically monitor these variables is highly desirable for both economic and quality reasons. Accurate monitoring can greatly reduce energy consumption caused by overheating and material consumption caused by an overtreatment. Other benefits of a continuous monitoring include the ability to measure high temperature phase changes, chemical reactions, and other related phenomena.

Methods and devices to determine these process relevant parameters are known in the field, mostly involving at least the use of a disposable probe carrying a sensor. Typically, the probe is brought under the surface of the melt in form of a drop-in sensor or by means of a lance assembly. The lance assembly can be operated manually, fully or semi-automated. The probe is connected to a processing device for processing the recorded data, typically by wires or cables, but also wireless data transfer has been described. Data is gathered and processed at or near real-time and provide the operator of the metallurgical facility with critical information about the progress or status of the metal making process occurring in the vessel.

For measurements with drop-in sensors, the disposable probe is dropped into the vessel containing the melt. Suitable probes are disclosed for example in EP 0758445 A1 and EP 0997716 A1. Such probes comprise a measuring head made from metal, which is mounted at one end of a carrier tube. The measuring head is usually made of cast iron or steel in order to provide the mass necessary to penetrate the slag layer built up on molten steel or iron. A signal cable connected to a measurement system is coiled inside the carrier tube and uncoils from the carrier tube as it falls into the molten metal. A sensor unit comprising at least one sensor, for example a temperature sensor and/or an electrochemical element for measurement of the oxygen activity of the molten metal, is located in the measuring head. The typical response time of commonly used sensors is in the range of 5 to 10 s. As the response time is the determining factor for the demand on all other components of the probe, a reduction of the response time is desirable.

Probes which are applied in BOF vessels (basic oxygen furnaces) typically have a mass of several kilograms and hold up to 30 m of cable. The cables used are chosen and adapted to survive the required travel time and to withstand the molten metal at least for the required response time of the sensor. Cables used for metallurgical applications are capable to withstand this environment for more than 10 s and have an outer diameter between 9 and 10 mm. The cables contain 2-3 wires, electrically insulated from each other and covered with an outer insulating layer made of a rubber material with an insulating layer thickness of 2,5 to 3 mm. The space required for the cable determines the dimensions of the carrier tube and also the mass of the measuring head. This mass needs to be sufficient to pull the cable out or from the tube and to gain enough speed to travel into the metal bath without an increase in travel time. Altogether, such probes have a mass between 6 to 8 kg, of which the measuring head (which is the assembly which is immersed during a measurement sequence) accounts for about 50 %. This material demand results in high material costs related to these probes and a reduction of this cost factor is desirable.

In current practice, the sensors are introduced from a position above the vessel containing the molten metal from a relatively large height, typical in the range of 10 to 20 m above the level of the molten metal. Several probes can be stored in a magazine and one probe at a time is released from the magazine for each measurement. The probe falls in free fall, accelerated by gravity, and sinks into the molten metal. The final speed of the probe when arriving at the molten metal surface is therefore determined by the distance between the drop station and the molten metal. The probes need to have a certain mass in order to sink deep enough under the melt surface to obtain reliable measurement data. Further components to provide a balance of the probe, like balancing bodies, which are not related to the recording of the measurement itself are required in order to provide reliable data or enable the measurement. Therefore, drop-in sensors introduce a relatively large amount of extra contaminating material into the to be measured molten material. Furthermore, the immersion point is not reliably controllable.

A molten metal is typically covered with a slag layer during its production, hereby subjecting any probe or sensor passing through it to increased harsh conditions, regardless of the specific methodology utilized. Thus, it is desired to minimize the duration of exposure as much as possible.

Especially in the field of electric arc furnaces (EAFs), there are presently only limited methods available to determine the parameters of the molten metal which can be conducted during the operation of the vessel. EAFs produce steel by using an electric arc to melt one or more charges of scrap metal, hot metal, iron based materials, or other meltable materials, which are placed within the furnace. In procedures common in EAFs, an operator manually inserts a lance carrying a suitable sensor into the furnace through the slag door, which is a relatively wide opening in the wall of the furnace shell. Such an invasive step is highly undesirable since it disturbs the environment within the vessel during the metallurgical process. Furthermore, energy is wasted caused by the intake of cold ambient air through the slag door when it is opened. Due to the design of an EAF with the electrodes positioned above the metal bath, standard drop-in probes cannot be applied in these facilities.

Recent developments have led to the availability of sensors with lower response times, for example in the form of needle shaped oxygen sensors. However, with the state-of-the art design of drop-in sensors, the improvement achieved cannot be utilized in the probes. In view of the prior art, there is the need for an improved measuring probe, which has a shorter response time. Furthermore, the probe shall have a simplified design to allow for a low-cost manufacturing.

The objective of the invention is thus to provide an improved measuring probe for measuring at least one parameter of a molten metal which solves at least one of the problems as described above. In particular, one of the objectives is to provide an improved measuring probe with reduced response time.

An additional aspect of the objective of the present invention is to provide a measuring probe which allows a simplification of the hardware required to utilize the probe.

An additional objective of the present invention is to provide a measuring probe which allows to be accelerated before a measurement is conducted.

Furthermore, it is an objective to provide a measuring probe which can be introduced to the measuring point through available entry points of a metallurgical vessel, in particular in an electric arc furnace (EAF).

Another aspect of the invention is to provide a method for measuring at least one parameter of a molten metal or slag with an inventive measuring probe, which allows the determination of the parameter with reduced effort and expense in terms of equipment, control technology, and organization, while at the same time achieving increased reliability and quality of the obtained measurements.

These objects are attained by the subject-matter defined in the independent claims.

The invention provides a measuring probe for a molten metal, comprising
- a sensor unit adapted to determine at least one parameter of the molten metal,
   wherein the sensor unit comprises
   a sensing element and
   a metal body, at least partly surrounding the sensing element;
- a signal line comprising at least two individual wires connected to the sensor unit; and
- a carrier element,
   characterized in that
   the inner diameter of the carrier tube is between 7 to 20 times the outer diameter of the individual wires.

Surprisingly, it has been found that a measuring probe with individual wires and an optimized ratio between the wire diameter and the dimensions of the carrier show an improved unwinding of the signal line when a measurement is taken. Furthermore, the probes are especially suitable to be accelerated and show stabilized flight characteristics. Both factors have surprisingly been observed to lead to a more reliable measuring probe. Furthermore, the individual wires allow a minimized design of the sensor unit, which can be accelerated and/or immersed without an excessive force on the signal line.

It is to be understood, that the inventive measuring probe is not intended to be used in conjunction with a lance, i.e. the probe shall not be immersed by an auxiliary item under the surface of a molten metal. The inventive measuring probe is in particular suitable to be provided at an entry point of a metallurgical vessel from where the part or parts which need to be immersed in the molten metal to obtain a measurement, in particular the sensor unit, is either dropped or actively accelerated towards to molten metal.

The measuring probe can be used in almost all metallurgical vessels. Especially in EAFs, the reduced size offers the further advantage that available entry points can be used, and an opening of the slag-door is not needed to obtain a measurement. Furthermore, it is possible to minimize the interruptions of a metallurgical facility for obtaining required parameter(s), in particular the method is applicable during a continuous operation. This minimizes the total operating costs, in particular the required energy input, and increases the throughput of the metallurgical facility as well as the quality of the products produced.

The invention relates to a measuring probe for a molten metal. A molten metal typically has a temperature above 600 °C, in particular above 800 °C, preferably above 1000 °C. The temperature of the molten metal can for example lie in the range of 600 - 1800 °C, more preferably in the range of 800 - 1700 °C.

Preferably, the molten metal is a molten steel. The term "melt" or "molten metal" does not exclude the presence of any solid or gaseous parts, including for example non-molten parts of the respective metal. The temperature of metal melts differs and usually depends on the composition of the metal and the stage of the melting process.

The molten metal may be covered with a slag layer. The term "slag" refers to non-steel byproducts that are often produced in a steel making furnace and are typically present as a molten material that floats on top of the molten metal. Slag may comprise metal oxides, metal sulfides, calcium oxide, magnesium oxide, magnesite, dolomite, iron oxide, aluminum oxide, manganese oxide, silica, sulfur, phosphorous, or a combination thereof. In order to obtain reliable measurements, a sensor introduced into the melt should pass through the slag layer as fast as possible to minimize thermal and corrosive effects and the freezing of the slag material on the cold sensor unit prior to reaching the final point of measurement in the molten metal. Such a frozen layer needs to melt when the sensor finally reaches the molten metal before a reliable measurement can be taken, thus prolonging the time the sensor needs to withstand the decomposing environment of the molten metal.

The invention relates to a measuring probe. A measuring probe is to be understood as a device which carries a sensor unit which can be at least partly immersed into a liquid of which a parameter is to be determined, the liquid of interest with regard to the present invention is a molten metal, i.e. a liquid with a high temperature. Prior to the measurement, the sensor unit of the measuring probe needs to be brought under the surface of the molten metal. The method to immerse the sensor unit into the molten metal is not further restricted, it may for example be dropped from a stationary point above the molten metal or may be accelerated by suitable means additional to gravitational forces. The compact design of the inventive measuring probe is compatible with different methods, allowing a versatile application of the measuring probe. Preferably, the sensor unit is accelerated by more than gravity, for example by a pneumatic acceleration device.

A measurement sequence with an inventive measuring probe typically comprises the separation of the sensor unit from a carrier element and a subsequent immersion of the sensor unit into the molten metal of interest.

The measuring probe comprises a sensor unit adapted to determine at least one parameter of the molten metal. The sensor unit is in principle constructed as a disposable component, which dissolves or decomposes in the molten metal after the parameter of interest is determined. Parts of the sensor unit may dissolve already prior to or during the determination of the parameter.

The parameter can be a physical, chemical or metallurgical parameter, for example the temperature, the presence, activity and/or concentration of chemical compound, in particular the oxygen activity, the carbon content, the aluminum content or the chemical composition.

"Determining a parameter" may be used herein as a synonym for measuring a parameter. The parameter can be determined from a single point measurement or a multiple point measurement. The determination can comprise the determination of a single parameter or the combination of more than one parameter. For example, the determination can comprise the measurement of the oxygen activity or the temperature of the molten metal. The determination can also comprise the measurement of the oxygen activity and the temperature.

The sensor unit comprises a sensing element. It is to be understood, that the sensing element is adapted to determine the at least one parameter of the molten metal. The sensing element can for example be at least one selected from the group consisting of an electrochemical sensor, an electromagnetic sensor, an optical sensor, a thermoelectric sensor, a sensor for detecting an electrical voltage, a sensor for detecting an electrical current or a sensor for detecting an electrical resistance. The sensor unit may also comprise more than one sensing element, preferably a combination of any of the named sensing elements, allowing combined measurements of several parameters. "A sensing element" thus is to be understood as "at least one sensing element" within the present application.

A thermoelectric sensor is preferably provided as a thermocouple. As known to the skilled person, a thermocouple comprises two wires of different materials, also referred to as legs of the thermocouple, which are joined at one end, typically referred to as hot junction. Such a thermocouple may be provided in a protecting element, for example in a tube, preferably in a quartz glass sheath. Depending on the type of thermocouple, such a quartz glass sheath may for example be at least one tubular or u-shaped quartz glass sheath.

In preferred embodiments, the thermocouple is a quartz glass sheathed thermocouple. The quartz glass sheathed thermocouple may comprise an outer closed end tube and an inner open-end tube arranged within the outer closed end tube, preferably both tubes are quartz glass tubes. The first leg of the thermocouple is arranged within the inner open-end tube and the second leg of the thermocouple is arranged in a hollow space between the inner open-end tube and the outer closed-end tube.

In an alternative embodiment, the thermocouple is provided without a sheath. In such cases, the thermocouple is preferably at least partly coated with a refractory material.

An electrochemical cell typically comprises a solid electrolyte material, a reference material, and an electrode. The electrochemical cell, in particular an electrochemical cell to determine the oxygen activity, may comprise a solid electrolyte tube, which is closed on one end, and which contains a reference material and an electrode at the closed end. Such sensors are for example disclosed in US 2002100686 A1. The electrochemical cell may also be provided as a needle sensor, which comprises a conductive wire which serves as an electrode with at least a solid electrolyte coating and a reference material coating. Such sensors are for example disclosed in US 5332449 A.

Preferably, the sensor unit comprises a thermocouple for measuring the temperature of the molten metal and/or an electrochemical cell, preferably an electrochemical cell for determining the oxygen activity of the molten metal.

The sensor unit may comprise a bath contact. A bath contact is to be understood as an electrically conductive means which provides an electrical contact between the sensor unit and the molten metal. The bath contact may be made from a metal, for example from molybdenum (Mo) or steel. The bath contact may be ring or rod shaped, preferably, the bath contact is ring-shaped.

A preferred sensor unit comprises a needle sensor to determine the oxygen activity, a thermocouple, preferably a thermocouple enclosed in a quartz glass sheath, and a ring-shaped bath contact, which surrounds the thermocouple. Such a sensor unit has a compact and robust design, which allows the miniaturization of a measuring probe carrying it.

Another preferred sensor unit comprises a needle sensor to determine the oxygen activity, a thermocouple with a refractory coating, and a ring-shaped bath contact, which surrounds the needle sensor and the thermocouple. Such a sensor unit has a compact and robust design, which allows the miniaturization of a measuring probe carrying it.

Preferably, the sensor unit is configured to provide at least one signal to a processing unit. Such a processing unit is configured to process the at least one signal and determine the parameter of the molten metal.

Preferably, the sensing element has a response time below 5 s, more preferred below 3 s. If the sensor unit comprises more than one sensing element, it is in particular preferred that all sensing elements have a response time below 5 s, more preferred below 3 s. The response time is the time which is required until a constant and stable measurement signal can be obtained after the sensing element has been introduced into the molten metal of which a parameter shall be determined. Sensing elements according to the state of the art are for example thermocouples with a typical response time in the range of 6 to 8 s in molten steel or electrochemical cells to determine the oxygen activity with a typical response time in the range of 8 to 10 s in molten steel. The utilization of a sensing element with a faster response time therefore allows to utilize components with a reduced mass and shielding.

The response time of the sensing element is mainly determined by the heat capacity of its active region. A sensing element with a small active region has a small heat capacity in this region, which leads to a short response time. The active region of the sensing element is to be understood as the section of the sensing element where the required signal is generated. For example, in case of a thermocouple the active region is the hot junction, in case of a needle shaped oxygen sensor, the active region is the tip of the needle. In preferred embodiments, the active region of the sensing element has a diameter of less than 2,5 mm, preferably less than 2 mm. For example, the active region of the sensing element may have a diameter in the range of 0,3 mm to 2,5 mm, preferred from 0,5 mm to 2 mm.

The sensor unit comprises a metal body, at least partly surrounding the sensing element. The metal body is provided by means of a sheathing in such a manner that the sensing element remains operable during the immersion of the immersion probe and for the duration of the measurement. Furthermore, the metal body aligns the sensor unit once it is immersed such that the sensing element has a suitable orientation and measurement position for the determination of the parameter of interest. Additionally, the metal body preferably surrounds a connection between the sensing element and the signal line, providing further protection of these sensitive and critical components of the sensor unit.

The metal body has two ends: The end of the metal body at which the sensing element is positioned is referred to as the immersion end, the opposite side along a longitudinal axis of the metal body is referred to as the rear end. The metal body at least partly surrounds the sensing element. In other words, the sensing element extends partly outward from the immersion end of the metal body.

Preferably, the metal body is made from a material with a high heat-capacity. In preferred embodiments, the metal body is made from steel, stainless steel, cast iron or copper. Before a reliable measurement can be taken, the active region of the sensing element needs to reach a thermal equilibrium with the molten metal and the metal body needs to provide long enough protection until this point can be reached. Most preferred, the metal body is at least partly made from copper. Copper has a high thermal conductivity; thus, the thermal equilibrium can be reached faster which enables a fast measurement. Copper is usually not considered as a suitable material for such metal bodies, since it contaminates the melt to be processed. In case of the present invention, the metal bodies can be miniaturized in such a way, that the introduced contamination can be neglected.

Preferably, the mass of the sensor unit is less than 500 g, more preferred less than 400 g, even more preferred less than 300 g, most preferred less than 200 g. The mass of the sensor unit is to be understood as the combined mass of the sensing element and the metal body and optional further components of the sensor unit. In other words, the mass of the sensor unit refers to all components of the sensor unit which are enclosed by the outer contour of the sensing element and the metal body. For example, the mass of the sensor unit is in the range of 80 to 500 g, more preferred in the range of 100 to 400 g, even more preferred in the range of 120 to 300 g, most preferred in the range of 140 to 200 g. The mass of the measuring head of drop-in sensors used nowadays including a metal body and the sensor elements lies in the range between 3 to 4 kg. A sensor unit with a significantly reduced mass minimizes the amount of material introduced into the molten metal during a measurement and additionally lowers production costs due to material savings. Furthermore, a measurement is only minimally influenced by the cold mass introduces by the lightweight sensor unit, allowing to obtain more reliable and exact data.

Preferably, the length of the metal body is not more than 70 mm, more preferably not more than 60 mm, even more preferably not more than 50 mm. The length of the metal body may for example be in the range of 20 to 70 mm, more preferred 30 to 60 mm. The length of the metal body is to be understood as the length along a central longitudinal axis from the immersion end to the rear end.

Preferably, the diameter of the metal body is not more than 40 mm, more preferred not more than 35 mm, even more preferred not more than 30 mm. The diameter of the metal body may for example be in the range of 10 to 40 mm, more preferred 15 to 35 mm. The diameter of the metal body is to be understood as the diameter perpendicular to a central longitudinal axis from the immersion end to the rear end.

Preferably, the metal body has a higher density than the density of the molten metal. Typically, molten steel has a density of approximately 7,0 g/cm³. Preferably, the density of the metal body is higher than 7,2 g/cm³, more preferred higher than 7,6 g/cm³. The density of the metal body may for example be in the range of 7,2 to 8,8 g/cm³, more preferred in the range of 7,6 to 8,6 g/cm³.

Preferably, the ratio of the mass of the sensor unit to the net density of the sensing element and the metal body is not more than 100 cm³, more preferred not more than 70 cm³, even more preferred not more than 50 cm³. For example, the ratio may be in the range of 15 to 100 cm³, more preferred in the range of 20 to 70 cm³ or 20 to 50 cm³. In drop in probes according to the state of the art with an average mass of 3500 g, this ratio is typically higher than 400 cm³. It has been found that a minimized ratio allows for sensor units with a compact design, enhancing the overall response time of the sensor unit provided by the sensing element.

In preferred embodiments, the density of the immersion end of the metal body is higher than the density on the rear end. Advantageously, the sensing element is oriented in a vertical direction when it is immersed in the molten metal. A density gradient of the metal body with a decreasing density towards the rear end supports such an orientation.

The metal body may be a monolithic component, it may also comprise more than one component. A modular design of the metal body allows to control the density distribution within the metal body and may facilitate its manufacture.

The metal body may be a hollow body with a tubular structure, preferably the metal body has a cylindrical structure which is symmetrical along a central longitudinal axis extending from the immersion end to the rear end. It is to be understood, that the metal body comprises a central void space.

Preferably, the largest cross-sectional area of the central void space of the metal body is smaller than 25 % of the largest total cross-sectional area of the metal body, more preferred smaller than 20 %. The cross-sectional area is to be understood as the area of a cross-section perpendicular to a central longitudinal axis from the immersion end to the rear end. A small area share of the void spaces ensures a sufficient density of the metal body.

The metal body may comprise more than one cross-sectional area along its length, for example, the metal body may comprise shoulders on the immersion end and/or on the rear end, in other words, the metal body may comprise recess sections at the outer circumference of its ends and thus a reduced cross-sectional area in this section or these sections.

The central void space may comprise a constant cross-sectional area along the length of the metal body, it may also comprise sections with different cross-sectional areas. For example, the central void space may comprise annular steps. Such a modular or stepped design of the void space may be advantageous for the assembly of the sensor unit and can secure the sensing element within the central void space. For example, the metal body may comprise a plurality of cylinders, which are arranged in line with each other in axial direction and which have inner bores with diameters becoming smaller towards the rear of the metal body.

The sensor unit may comprise a housing which encloses the sensing element or the more than one sensing elements. Preferably, the housing encloses the connection between the sensing element and the signal line. Such a housing may enhance the connection and additionally facilitate the assembly of the sensor unit. The housing may for example comprise a body of a refractory material, such as a refractory cement, a glue filling and/or a metal support.

In preferred embodiments, the sensing element, preferably the at least one sensing element enclosed in a housing, fills the void space of the metal body, in other words, the sensing element or the housing enclosing the at least one sensing element is at least partly shaped to fit in the central void space of the metal body. Thus, the compact design of the sensor unit can be further enhanced and voids with a low density are avoided. Additionally, no further filling elements are required, as disclosed for example in EP 0758445 A1 as a solution to increase the total density of a drop-in probe.

Preferably, the combined net density of all components enclosed by the outer contour of the sensing element and the metal body is higher than the density of the molten metal. The combined net density of all components enclosed by the outer contour of the sensing element and the metal body is to be understood as the combined density of these components, i.e. the density of the whole building block which may also comprise void space or further elements enclosed by the metal body, for example an electrical contact element or a housing of the sensing element. In the following, it will be referred to this parameter as the combined net density of the sensing element and the metal body for brevity. In preferred embodiments, the combined net density of the sensing element and the metal body is at least 5 % higher than the density of the molten metal, more preferred at least 8 % higher, even more preferred more than 10 % higher. The combined net density of the sensor unit and the metal body may be higher than 7,2 g/cm³, more preferably higher than 7,5 g/cm³, even more preferred higher than 7,8 g/cm³. The combined net density of the metal body may for example be in the range of 7,2 - 8,6 g/cm³, more preferred in the range of 7,6 - 8,4 g/cm³. In preferred embodiments, the combined net density of the sensing element and the metal body is at least 80 % of the density of the metal body, more preferred at least 90 %.

In preferred embodiments, the share of void spaces enclosed by the sensing element and the metal body is lower than 20 % with respect to the net outer volume of the sensing element and the metal body, more preferred lower than 15 %, most preferred lower than 10 %. Void spaces enclosed by the sensor unit are cavities with a density which is lower than 20 % of the combined net density of the sensing element and the metal body. The net outer volume the sensing element and the metal body is to be understood as the volume enclosed by the outer contour of the sensing element and the metal body.

Preferably, the sensor unit does not comprise an additional mass component or buoyant component. Sensor units with a low mass are especially suitable to be accelerated, thus they gain a high velocity prior to their immersion in the molten metal bath, which eliminates the need for such a further balancing. Thus, the sensor unit design can be simplified and miniaturized. This miniaturization further allows to introduce the sensor unit into metallurgical vessels through available entry points, for which conventional drop-in sensors or lance systems are too large.

The sensor unit may comprise an electrical contact element, which is adapted to connect the sensor unit to a suitable means to transfer the measured data, typically to the signal line. Such a contact piece allows an easy mounting of the sensor unit, stabilizes the contact to the signal line and reduces the forces on the sensing element. Preferably, a bath contact and the sensing element are electrically connected to the electrical contact element.

Advantageously, the electrical contact element is arranged completely within the metal body, in particular within the central void space of the metal body.

The measuring probe comprises a signal line connected to the sensor unit. The signal line is adapted to transfer a signal acquired by the sensor unit, in particular by the sensing element, to a suitable processing unit or a transfer means which is adapted to transfer the signal to such a processing unit. In other words, the signal line preferably provides a connection between the sensor unit and a processing unit.

Preferably, the signal line is connected to the rear end of the sensor unit, preferably by means of an electrical contact element. The rear end of the sensor unit is to be understood as the end at which the rear end of the metal body is positioned. The signal line extends from a portion which is connected to the signal unit, the connection section, to an opposite portion, the rear section.

The signal line comprises at least two individual wires, in other words the signal line comprises at least two single wires which are not enclosed in a common cable sheath. A wire in terms of the present invention is to be understood as an electrically conductive core, typically made from a metal material which is embedded in an insulating sheath, for example from a rubber or silicone material. The electrically conductive core may comprise a plurality of single fibers or filaments. A cable is to be understood as a plurality of wires which are enclosed in a common cable sheath. The utilization of individual wires has surprisingly been found to enhance the unwinding behavior of the signal line form the carrier element and furthermore leads to an improvement of the free-flight phase of the sensor unit once it its directed to a molten metal. Additionally, single wires have a reduced mass, thus allowing reducing the mass of the sensor unit, which needs to pull the signal line during the free flight.

The signal line must meet a number of requirements: It needs to withstand the measurement environment long enough until the sensor unit is brought to the point of measurement and until the required data is obtained. Furthermore, the signal line needs to be compatible with the method to immerse the sensor unit, for example to allow for an acceleration of the sensor unit. Especially in methods which comprise a free flying phase of the probe, the signal line should support the control of this phase without interference. Thus, the flexibility and material of the signal line need to be selected accordingly.

The metal material may be chosen from the group containing copper (Cu), copper-nickel (CuNi), chromel (nickel (Ni) based alloy with chromium (Cr)) and alumel (nickel (Ni) based alloy with aluminium (Al), manganese (Mn) und silicon (Si)). In preferred embodiments, the wire comprises a copper (Cu) or a copper-nickel (CuNi) conductive core. The cross-sectional area of the conductive core may be in the range of 0,05 to 3 mm², preferably 0,1 to 2,5 mm².

Suitable materials are for example rubbers, preferably silicone rubbers or EPDM (ethylene propylene diene monomer) rubbers. Preferably, the material of the insulating sheath does not melt completely when in contact with the molten material but degrades, preferably at least partly to an inorganic material. Such a degrading behavior allows for a longer protection of the conductive core of the wire and thus prolongs the lifetime of the signal line before its breakdown. Preferably, the insulating material is a silicone rubber.

Preferably, the individual wires have an outer diameter of less than 3 mm, more preferred less than 2 mm, even more preferred less than 1 mm. For example, the individual wires have an outer diameter in the range of 0,2 to 3 mm, more preferred 0,4 to 2 mm, even more preferred 0,3 to 1 mm. The outer diameter of the wire refers to the cross-sectional area perpendicular to the longitudinal length of the wire through the conductive core and the insulating sheath. Wires commonly used with state-of-the-art sensor units typically have a diameter larger than 4 mm, which leads to a relatively high rigidity. Wires which can be used with the sensor unit according to the invention may have a smaller diameter, leading to a higher flexibility. Such a flexibility has been shown to be advantageous for the stability of the connection of the signal line to the sensor unit. Furthermore, it allows are more compact design of the whole measuring probe, especially of the carrier element.

In preferred embodiments, the ratio of the diameter of the active region of the sensing element and the diameter of the individual wires is in the range of 1 - 1 to 1 - 4, preferably in the range of 1 - 2 to 1 - 3. A ratio in this region ensures that the individual wires are compatible with the response time of the sensing element.

Preferably, the density of the signal line is lower than the density of the molten metal. In such a configuration, the signal line generates a buoyant force when the sensor unit is immersed in the molten metal and supports a vertical alignment of the sensor unit during the measurement. Preferably, the density of the signal line is in the range of the density of the slag layer which typically covers a molten metal, which is in average around 1,8 g/cm³. Such a configuration results in a buoyant force of the part of the signal line which is immersed in the molten metal and a gravitational force in the opposite direction of the part which is located in the slag layer. Preferably, the density of the signal line is lower than 5 g/cm³, more preferred lower than 4 g/cm³. The density of the signal line may for example be in the range of 1 - 5 g/cm³, more preferred in the range of 2 - 4 g/cm³.

In preferred embodiments, the density of the signal line is lower than the combined net density of the sensing element and the metal body. It has been found that the density of the signal line advantageously corresponds to the combined net density of the sensing element and the metal body. A balance between the densities of these components improves the sinking and rising behavior of the sensor unit when it is immersed for a measurement in the molten metal and further supports a vertical alignment of the sensor unit during the measurement. Preferably, the density of the signal line is not higher than 50 % of the combined net density of the sensing element and the metal body, more preferred not higher than 40%, even more preferred not more than 30 %.

The signal line can comprise a single portion or a plurality of portions which are connected to each other. A portion is to be understood as a section which comprises the same materials.

It is to be understood that the length of the signal line is chosen to be long enough to bridge the distance of the measurement point of the sensor unit to the processing unit or at least to a connection means which transfers the signal of the sensor unit.

The sensor unit may comprise a steering element. The steering element provides balance of the sensor unit during a free flight phase during immersion and thus improves the behavior of these parts during this moving phase. Furthermore, the steering element may provide additional support for the signal line and ensures an organized unwinding of the signal line during a moving phase of the sensor unit during a measurement sequence.

The steering element is preferably provided on or at the rear end of the metal body of the sensor unit.

The steering element may be an elongated component, for example a wire shaped element. The steering element may be made from a metal, for example from copper, steel or stainless steel. In preferred embodiments, the steering element is a steel wire. The diameter of the wire may be in the range of 0,3 - 3 mm, preferably in the range of 0,8 - 2,5 mm.

The length of the steering element may be in the range of 5 to 25 cm, preferably in the range of 10 to 20 cm.

In preferred embodiments, a first section of the signal line is laid in at least one loop along the steering element. Such a looped guidance of the signal line provides for a certain overlength which moves with the signal unit as soon as it is in motion, which ensures a stable connection between signal line and sensor unit. Preferably, the length of the first section of the signal line is not more than 5 times the length of the steering element, preferably not more than 3 times.

The loop of the signal line may be fixated to the steering element, for example by a suitable fixing means as a clip or a paper tape. Preferably only one leg of the loop of the first section of the signal line is fixated to the steering element. Such a one-legged fixation allows for a more balanced movement of the sensor unit with the steering element during use.

Another aspect of the invention is a sensor assembly comprising a sensor unit with a steering element and a signal line connected to the sensing element of the sensor unit.

Aspects of the invention directed to the sensor unit, the steering element and the signal line of the inventive measuring probe described above are also preferred aspects of the inventive sensor assembly.

The measuring probe comprises a carrier element. The carrier element is preferably a hollow body, for example a tube, preferably a cardboard tube, which is configured to carry and/or accommodate the other components of the measuring probe. Such a carrier element protects and carries the other components of the measuring probe before use.

The signal line may be wound up within the carrier element. For example, the signal line can run through the inside of a carrier tube and be wound up inside of the carrier tube around its longitudinal axis in at least one layer. Preferably, the signal line is wound up in loops with a diameter in the range of 7 to 20 times the outer diameter of the individual wires, preferably in the range of 8 to 15 times. The diameter of the loop refers to the outer diameter of the loop. In case the signal line is wound up in more than one layer, the diameter refers to the outer diameter of the inner layer. The inner layer is to be understood as the layer with the largest distance to the inner surface of the carrier element.

The inner diameter of the carrier element is between 7 to 20 times the outer diameter of the individual wires of the signal line, preferably between 8 to 15 times the outer diameter of the individual wires. Surprisingly, it has been shown to be advantageous when the outer diameter of individual wires of the signal line corresponds to the inner diameter of the carrier element, especially when the carrier element is a tube. Such a ratio ensures a sufficient fixation of the signal line within the carrier element while still allowing a controlled uncoiling without the need for additional pulling forces.

Preferably the individual wires of the signal line have an outer diameter of less than 3 mm and the inner diameter of the carrier element is between 7 to 20 times the outer diameter of the individual wires. It has been found that such a correlation is advantageous for a compact design of the measuring probe while still ensuring a sufficient stability of the signal line organization within the carrier element.

Preferably, the hollow body of the carrier element has an inner diameter in the range of 20 to 60 mm, more preferred in the range of 25 to 50 mm.

The sensor unit is preferably separable from the carrier element. In other words, the sensor unit may be releasably mounted in or on the carrier element. In use, the sensor unit can be released from the carrier element and immersed into the molten metal while the signal line is pulled behind and out of the carrier element. It is to be understood, that the signal line is not released from the sensor unit but remains connected at least until a measurement sequence is concluded.

For example, the measuring probe may comprise a first coupling component which is configured to releasably engage a second coupling component arranged on or at the sensor unit. The measuring probe may also comprise a catch element, which releases the sensor unit upon the application of a certain force.

The sensor unit may be positioned at least partly in the carrier element. The end of the carrier element on or at which the sensor unit is positioned is referred to as the front end of the carrier element, the opposite end is referred to as the rear end of the carrier element.

The measuring probe may comprise a holding element, which enables a release mechanism for the sensor unit from the carrier element. The holding element may be fixed to the carrier element and be configured to releasably engage one end of the sensor unit. The holding element may comprise at least one opening for the signal line.

The measuring probe may comprise a fixing element arranged within the carrier element. The fixing element is configured to ensure a suitable fixation and organization of the signal line within the carrier element. Depending on the length of the signal line, the deacceleration of the signal line may need to be regulated. This can be realized by increasing the required pulling force to remove the last portion of the rear section of the signal line from its winding or windings. The fixing element additionally buffers the pulling forces from the signal line on the sensor unit and the connection element during the immersion of the probe.

Preferably, the fixing element is positioned at the rear end of the carrier element. In preferred embodiments, the fixing element is positioned on the signal line, in other words, the signal line at least partly extends between the inside of the carrier element and the fixing element. The fixing element may comprise an opening configured for a free passage of the signal line. Such an opening allows a free sliding through of the signal line when the windings are unwound in free fall.

The fixing element may for example be provided as a ring with an opening through which the signal line may extend.

The measuring probe may comprise a probe contact element which is adapted to be connected to the rear section of the signal line. Such an electrical connection element may preferably be adapted to be positioned in the carrier element, for example it may be a plug type element.

Preferably, the probe contact element is adapted to transfer the signal of the signal line to a processing unit, for example by wired or wireless transmission.

The measuring probe may comprise a protection element. Such a protection element protects the sensor unit during handling and against damage caused by impact on the molten metal surface. Preferably, the measuring probe comprises a protective cap, which encloses at least the sensor unit, and which is formed from a material that dissolves or melts in the molten metal. The protection element may for example be made from aluminum or copper, preferably from copper.

The protection element is preferably provided on or at the immersion side of the sensor unit, preferably on the immersion end of the metal body of the sensor unit. Thus, the protection element is the first part of the sensor unit which contacts the molten metal when the sensor unit is immersed.

The protection element may have a conical or frusto-conical shape. A conical or frusto-conical shape on the one hand side improves the flying behavior of the immersion probe and on the other side reduces frictional forces when the probe is immersed in the molten metal.

The protection elements may comprise openings, for example circular, elliptical, or slit shaped openings. Such openings enhance the melting behavior of the protection element, especially in the case of protection elements provided as metal caps.

In another aspect, the present invention relates to a method for measuring at least one parameter of a molten metal or slag with an inventive measuring probe. The method may comprise the following steps:
i) providing the measuring probe;
ii) separating the sensor unit from the carrier element;
iii) immersing the sensor unit in the molten metal;
iv) measuring the at least one parameter of the molten metal.

The steps of the method are conducted in the given consecutive order.

Aspects of the invention directed to the inventive measuring probe described above are also preferred aspects of the inventive method.

It has been found that the inventive measuring probe is especially suitable for an additional acceleration. Surprisingly, an acceleration of the sensor unit by more than only gravity offers several advantages. The probe accommodating the sensor unit can be reduced in terms of mass, dimensions and number of components. The reduced material demand results in a cost reduction and a reduction of contaminating material introduced into the molten metal. This miniaturization of the sensor and the measuring probe in turn allows to introduce the sensor unit through openings with a reduced size which were previously not usable for the introduction of probes with state-of-the-art methods.

The method according to the invention comprises separating the sensor unit from the carrier element. In other words, the sensor unit is released from the carrier element of the measuring probe prior to the immersion of the sensor unit. This separation may for example be realized by a releasing mechanism provided with the measuring probe and/or corresponding releasing means provided by an external device.

The method according to the invention comprises immersing the sensor unit in the molten metal, in other words the sensor unit enters the molten metal through its surface. In cases of molten metals with a present slag layer, the sensor unit will move through this slag layer before entering the molten metal.

The method comprises measuring the at least one parameter of the molten metal. It is to be understood, that the measurement is taken when the sensor unit is immersed under the surface of the molten metal. "Measuring" is used herein to describe a step resulting in the determination of the at least one parameter. This step may comprise the measurement of a single data point or the measurement of more than one data point; i.e. the measurement of a series of data points. The measuring may comprise additional steps, for example the transmittance of data to a processing unit and/or the processing of the data.

The method may comprise further steps. For example, the method may comprise a step during which the sensor unit is accelerated towards the molten metal by suitable acceleration means, for example by means of a pneumatic accelerator.

After the measuring of the parameter, further steps may follow, for example the release or ejection of parts of the measuring probe, the cutting of remaining cables or the like.

The following schematic drawings show aspects of the invention for improving the understanding of the invention in connection with some exemplary illustrations. It should be understood, however, that the invention is not limited to the precise arrangements and instrumentalities shown. The elements of the drawings are not necessarily to scale relative to each other. Like reference numerals designate corresponding similar parts. Herein
- Figure 1: shows a schematic cross-sectional view of a measuring probe;
- Figure 2: shows a sensor unit in more detail;
- Figure 3: shows an exemplary set up for a measurement with a measuring probe;
- Figure 4: shows a sensor assembly with sensor unit which comprises a steering element.

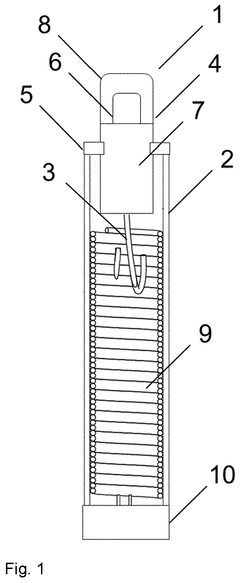

Figure 1 shows a cross-sectional view of a measuring probe 1. The probe 1 comprises a cardboard tube as a carrier element 2 in which a signal line 3 is wound up. A sensor unit 4 is mounted at least partly within the carrier tube 2 at one end and is held by a releasing mechanism 5. Within the sensor unit 4, a sensing element 6 is embedded in a copper body 7. To protect the sensing element 6 during the handling of the measuring probe 1, a protective cap 8 made of a material that dissolves or melts in the molten metal encloses the sensing element 6. The protective cap 8 may for example be made from copper. Along the inside of the carrier tube 2 the signal line 3 is wound in windings 9. One end of the signal line 3 is connected to the sensor unit 4, the other end of the signal line 3 is connected to a probe contact element 10 at the rear end of the carrier tube 2. The probe contact element 10 may provide a suitable connection point to an extension cable or means to wirelessly transfer a signal acquired by the sensor unit 4 to a processing device.

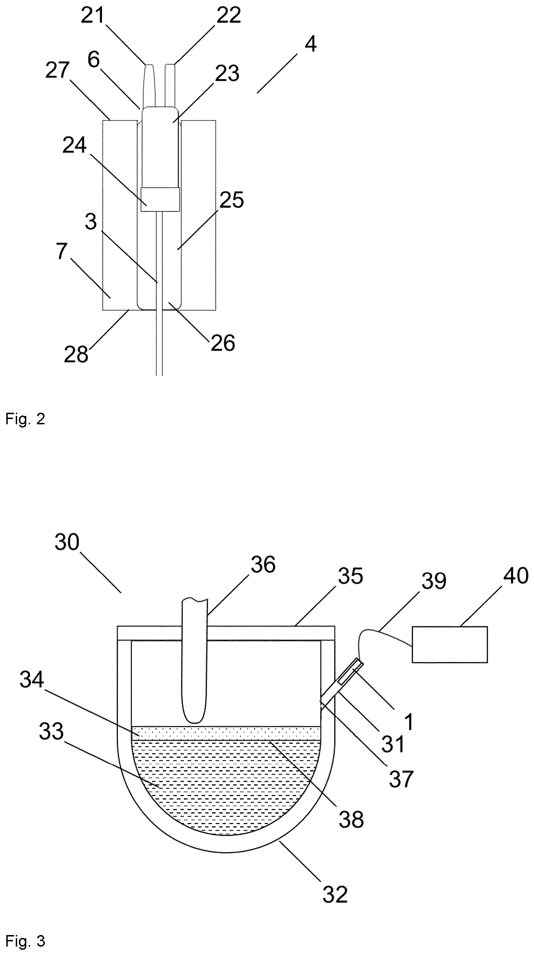

Figure 2 shows a cross-sectional view of a sensor unit 4 in more detail. The sensor unit 4 comprises a needle shaped oxygen measurement cell 21 and a thermocouple 22 as sensing elements. The sensor unit 4 is therefore able to measure the oxygen activity and the temperature of a molten metal when it is brought in contact with a metal melt. The needle cell 21 is for example a molybdenum (Mo) pin, which is coated with a layer of a mixture of chromium and chromium dioxide (Cr-Cr₂O₃) as reference material under a layer of stabilized zirconium oxide (stabilized zirconia) as solid electrolyte. The diameter of the tip of the oxygen sensor is in the range of 0,5 to 2 mm. Suitable thermocouples are for example type B, type D, type G or type C thermocouples, of which type C thermocouples, which comprise legs of tungsten-rhenium (W/Re) alloys (95 wt.-% W/ 5 wt.-% Re vs. 74 wt.-% W/ 26 wt.-% Re), are preferred. At the hot junction of the thermocouple, the diameter is not larger than 2 mm to ensure a fast response time. Both sensing elements (21, 22) are enclosed in a ring-shaped bath-contact 23. The sensing elements (21, 22) are connected to an electrical connector 24 to which the signal line 3 is also connected. The connection of the sensing elements (21, 22) to the electrical connector 24 may be secured by a housing 25 of refractory cement, any other suitable refractory material or partly by a suitable glue. The outer shape of the housing 25 of the sensing elements (21, 22) is generally cylindrically shaped and sized to snugly fit within the bore 26 of the metal body 7. The central longitudinal bore 26 extends from the immersion end 27 to the rear end 28 through the body 7 and can for example be drilled in a cast solid metal body. The mass of the complete assembly is around 180 g with a density of 8 g/cm³, of which 150 g are related to the metal body.

During a measurement sequence, the measuring probe will be positioned above a molten metal bath. When in place, the release and separation of the sensor unit from the carrier can be initiated by suitable means. Subsequently, the sensor unit will move towards the molten metal, either accelerated only by gravity or by a further external acceleration mechanism.

Figure 3 shows an exemplary set up for a measurement in which the inventive measuring probe 1 may advantageously be used. In the side wall of a metallurgical vessel 30 like an electrical arc furnace (EAF) an acceleration means (an accelerator, 31) is integrated. An EAF used for steelmaking usually comprises a container 32 holding the molten metal bath 33 and a removable lid 35 through which one or more electrodes 36 can enter the furnace. A slag layer 34 covers the molten metal 33. The electrodes 36 employed to heat the metal are arranged above the container 32. Typically, the interior of the metallurgical vessel 30 is heated to temperatures of about 600 - 2000 °C or even higher during processing.

When a measurement shall be conducted in an installation as shown in Fig. 3, the accelerator 31 is loaded in a first step with the measuring probe 1 (configuration shown). An extension cable 39 connects the sensor unit of the measuring probe 1 to a processing device 40 which may be placed in a distance to the vessel 30.

Inside the accelerator 31, the sensor unit is separated from the carrier parts of the probe 1. This separation may for example be realized by a suitable installation inside the accelerator 31, like a shoulder or a barrel shaped cone, against which a holding means of the probe 1 is pushed to release the sensor unit. It shall be emphasized, that any connections between the sensor unit and a signal line or suitable connectors are not released and are all configured to remain in place at least until the measurement sequence is completed.

Subsequently, the sensor unit is accelerated, for example by compressed air, and ejected from the accelerator 31 into the interior of the vessel 30 and towards the molten metal bath 33 with a high initial speed and momentum. The sensor unit flies on a straight path towards the molten metal 33 and penetrates the surface 38. The signal line which is connected to the sensor unit will be pulled behind the sensor unit and out of the carrier element and is chosen to survive the harsh environment inside the vessel long enough to ensure that the measurement can be taken.

Due to the individual wires, the related minimized size and mass and optimized dimensional ratios, a measuring probe according to the invention is especially suitable for a measurement sequence with an active acceleration.

When the sensor unit is immersed under the surface of the molten metal bath, the desired parameter can be measured, and the respective signal is transferred to a suitable processing device 40. After the recording of the required data, the accelerator 31 may be cleared from the elements of the probe 1 which have not been projected into the molten metal, for example by ejecting them into the molten metal bath 33.

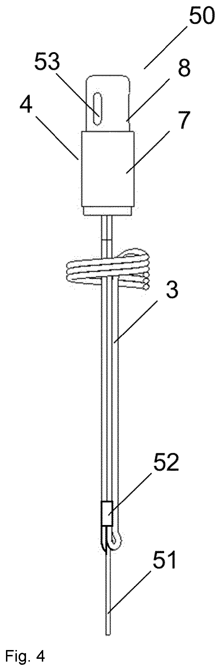

Fig. 4 shows a sensor assembly 50 with sensor unit 4 which comprises a steering element 51. The steering element 51 supports the first section of the signal line 3 which is connected to the sensing elements within the metal body 6 (connection not shown). The steering element 51 is wire shaped, it may for example be a steel wire, with its first end attached to the metal body 7 of the sensor unit 4. In the shown embodiment, the signal line 3 comprises three individual wires. The wires are routed along the steering element 51 in a single elongated loop and are partially fixated to it. The fixation means may for example be a paper tape 52, which burns away easily as soon as the sensor unit 4 enters the molten metal. The sensor assembly 50 comprises a cup-shaped protection element 8 with an elongated lateral slit 53. Such an opening accelerates the melting of the protection element 8 in the molten metal. The cap may comprise more than one lateral opening.

It will be appreciated by those skilled in the art that changes or modifications could be made to the above-described embodiment without departing from the broad inventive concepts of the invention. It should be appreciated, therefore, that the present invention is not limited to the particular embodiment disclosed but is intended to cover all embodiments within the scope of the appended claims.

### Reference Signs

- 1: measuring probe
- 2: carrier element
- 3: signal line
- 4: sensor unit
- 5: releasing mechanism
- 6: sensing element
- 7: metal body
- 8: protective cap
- 9: windings of signal line
- 10: probe contact element
- 21: oxygen measurement cell
- 22: thermocouple
- 23: bath contact
- 24: electrical connector
- 25: housing of sensing elements
- 26: bore of metal body
- 27: immersion end of metal body
- 28: rear end of metal body
- 30: metallurgical vessel
- 31: accelerator
- 32: container
- 33: molten metal bath
- 34: slag layer
- 35: removable lid
- 36: electrode of EAF
- 37: entry point
- 38: surface of molten metal bath
- 39: extension cable
- 40: processing device
- 50: sensor assembly
- 51: steering element
- 52: paper tape
- 53: opening in protection element

## Claims

1. A measuring probe (1) for a molten metal, comprising
- a sensor unit (4) adapted to determine at least one parameter of the molten metal,
wherein the sensor unit (4) comprises
a sensing element (6) and
a metal body (7), at least partly surrounding the sensing element (6);
- a signal line (3) comprising at least two individual wires connected to the sensor unit (4); and
- a carrier element (2),
**characterized in that**
the inner diameter of the carrier element (2) is between 7 to 20 times the outer diameter of the individual wires.

2. A measuring probe (1) according to claim 1, wherein the individual wires have an outer diameter in the range of 0,2 to 3 mm.

3. A measuring probe (1) according to claim 1 or 2, wherein the signal line (3) is wound up within the carrier element (2).

4. A measuring probe (1) according to any of the preceding claims, wherein the mass of the sensor unit (4) is in the range of 80 to 500 g.

5. A measuring probe (1) according to any of the preceding claims, wherein the sensor unit (4) comprises more than one sensing element (6).

6. A measuring probe (1) according to any of the preceding claims, wherein the sensor unit (4) comprises a thermocouple (22) and/or an electrochemical cell (21).

7. A measuring probe (1) according to any of the preceding claims, wherein the active region of the sensing element (6) has a diameter of less than 2,5 mm.

8. A measuring probe (1) according to any of the preceding claims, wherein the ratio of the diameter of the active region of the sensing element (6) and the diameter of the individual wires is in the range of 1 - 1 to 1 - 4.

9. A measuring probe (1) according to any of the preceding claims, wherein the at least one sensing element (6) has a response time below 5 s.

10. A measuring probe (1) according to any of the preceding claims, wherein the combined net density of the sensing element (6) and the metal body (7) is at least 80 % of the density of the metal body (7).

11. A measuring probe (1) according to any of the preceding claims, wherein the largest cross-sectional area of a central void space (26) of the metal body (7) is smaller than 25 % of the largest total cross-sectional area of the metal body (7).

12. A measuring probe (1) according to any of the preceding claims, wherein the density of the signal line is not higher than 50 % of the combined net density of the sensing element (6) and the metal body (27).

13. A measuring probe (1) according to any of the preceding claims, wherein the sensor unit (4) comprises a steering element (51).

14. A method for measuring at least one parameter of a molten metal or slag with a measuring probe (1) according to any of claims 1 - 13.

## Patentansprüche

1. Messsonde (1) für eine Metallschmelze, umfassend
- eine Sensoreinheit (4), die angepasst ist, um mindestens einen Parameter der Metallschmelze zu bestimmen, wobei die Sensoreinheit (4) umfasst
ein Sensorelement (6) und
einen Metallkörper (7), der das Sensorelement (6) mindestens teilweise umgibt;
- eine Signalleitung (3), umfassend mindestens zwei einzelne Drähte, die mit der Sensoreinheit (4) verbunden sind; und
- ein Trägerelement (2),
**dadurch gekennzeichnet, dass**
der Innendurchmesser des Trägerelementes (2) zwischen dem 7- bis 20-Fachen des Außendurchmessers der Einzeldrähte beträgt.

2. Messsonde (1) nach Anspruch 1, wobei die einzelnen Drähte einen Außendurchmesser in dem Bereich von 0,2 bis 3 mm aufweisen.

3. Messsonde (1) nach Anspruch 1 oder 2, wobei die Signalleitung (3) innerhalb des Trägerelements (2) aufgewickelt ist.

4. Messsonde (1) nach einem der vorstehenden Ansprüche, wobei die Masse der Sensoreinheit (4) in dem Bereich von 80 bis 500 g liegt.

5. Messsonde (1) nach einem der vorstehenden Ansprüche, wobei die Sensoreinheit (4) mehr als ein Tastelement (6) umfasst.

6. Messsonde (1) nach einem der vorstehenden Ansprüche, wobei die Sensoreinheit (4) ein Thermoelement (22) und/oder eine elektrochemische Zelle (21) umfasst.

7. Messsonde (1) nach einem der vorstehenden Ansprüche, wobei der aktive Bereich des Tastelements (6) einen Durchmesser von weniger als 2,5 mm aufweist.

8. Messsonde (1) nach einem der vorstehenden Ansprüche, wobei das Verhältnis des Durchmessers des aktiven Bereichs des Tastelements (6) und des Durchmessers der einzelnen Drähte in dem Bereich von 1 - 1 bis 1 - 4 liegt.

9. Messsonde (1) nach einem der vorstehenden Ansprüche, wobei das mindestens eine Sensorelement (6) eine Ansprechzeit unter 5 s aufweist.

10. Messsonde (1) nach einem der vorstehenden Ansprüche, wobei die kombinierte Nettodichte des Sensorelementes (6) und des Metallkörpers (7) mindestens 80 % der Dichte des Metallkörpers (7) beträgt.

11. Messsonde (1) nach einem der vorstehenden Ansprüche, wobei die größte Querschnittsfläche eines zentralen Hohlraums (26) des Metallkörpers (7) kleiner als 25 % der größten Gesamtquerschnittsfläche des Metallkörpers (7) ist.

12. Messsonde (1) nach einem der vorstehenden Ansprüche, wobei die Dichte der Signalleitung nicht höher als 50 % der kombinierten Nettodichte des Sensorelements (6) und des Metallkörpers (27) ist.

13. Messsonde (1) nach einem der vorstehenden Ansprüche, wobei die Sensoreinheit (4) ein Lenkungselement (51) umfasst.

14. Verfahren zum Messen mindestens eines Parameters einer Metallschmelze oder Schlacke mit einer Messsonde (1) nach einem der Ansprüche 1 - 13.

## Revendications

1. Sonde de mesure (1) pour un métal en fusion, comprenant
- une unité de capteur (4) adaptée pour déterminer au moins un paramètre du métal en fusion, dans laquelle l'unité de capteur (4) comprend
un élément de détection (6) et
un corps métallique (7) entourant au moins partiellement l'élément de détection (6) ;
- une ligne de signal (3) comprenant au moins deux fils individuels connectés à l'unité de capteur (4) ; et
- un élément porteur (2),
**caractérisée en ce que**
le diamètre intérieur de l'élément porteur (2) est compris entre 7 et 20 fois le diamètre extérieur des fils individuels.

2. Sonde de mesure (1) selon la revendication 1, dans laquelle les fils individuels ont un diamètre extérieur compris entre 0,2 et 3 mm.

3. Sonde de mesure (1) selon la revendication 1 ou 2, dans laquelle la ligne de signal (3) est enroulée à l'intérieur de l'élément porteur (2).

4. Sonde de mesure (1) selon l'une quelconque des revendications précédentes, dans laquelle la masse de l'unité de capteur (4) est comprise entre 80 et 500 g.

5. Sonde de mesure (1) selon l'une quelconque des revendications précédentes, dans laquelle l'unité de capteur (4) comprend plus d'un élément de détection (6).

6. Sonde de mesure (1) selon l'une quelconque des revendications précédentes, dans laquelle l'unité de capteur (4) comprend un thermocouple (22) et/ou une cellule électrochimique (21).

7. Sonde de mesure (1) selon l'une quelconque des revendications précédentes, dans laquelle la zone active de l'élément de détection (6) a un diamètre inférieur à 2,5 mm.

8. Sonde de mesure (1) selon l'une quelconque des revendications précédentes, dans laquelle le rapport entre le diamètre de la zone active de l'élément de détection (6) et le diamètre des fils individuels est compris entre 1:1 et 1:4.

9. Sonde de mesure (1) selon l'une quelconque des revendications précédentes, dans laquelle l'au moins un élément de détection (6) a un temps de réponse inférieur à 5 s.

10. Sonde de mesure (1) selon l'une quelconque des revendications précédentes, dans laquelle la densité nette combinée de l'élément de détection (6) et du corps métallique (7) est d'au moins 80 % de la densité du corps métallique (7).

11. Sonde de mesure (1) selon l'une quelconque des revendications précédentes, dans laquelle la plus grande surface de section transversale d'un espace vide central (26) du corps métallique (7) est inférieure à 25 % de la plus grande surface de section transversale totale du corps métallique (7).

12. Sonde de mesure (1) selon l'une quelconque des revendications précédentes, dans laquelle la densité de la ligne de signal n'est pas supérieure à 50 % de la densité nette combinée de l'élément de détection (6) et du corps métallique (27).

13. Sonde de mesure (1) selon l'une quelconque des revendications précédentes, dans laquelle l'unité de capteur (4) comprend un élément de direction (51).

14. Procédé de mesure d'au moins un paramètre d'un métal en fusion ou d'un laitier à l'aide d'une sonde de mesure (1) selon l'une quelconque des revendications 1 à 13.
